Europäisches Patentamt

(19) European Patent Office

Office européen des brevets

(11) Publication number: **0 173 327 B1**

(12) **EUROPEAN PATENT SPECIFICATION**

(45) Date of publication of patent specification: **06.11.91**

(21) Application number: **85110856.3**

(22) Date of filing: **28.08.85**

(51) Int. Cl.⁵: **C12N 15/31, C12N 1/21,**
**C12P 21/00, //(C12N1/21,**
**C12R1:19)**

(54) Bialaphos producing gene.

(30) Priority: **30.08.84 JP 181151/84**

(43) Date of publication of application:
**05.03.86 Bulletin 86/10**

(45) Publication of the grant of the patent:
**06.11.91 Bulletin 91/45**

(84) Designated Contracting States:
**CH DE FR GB LI**

(56) References cited:
**EP-A- 0 094 506**
**EP-A- 0 118 367**

**THE JOURNAL OF ANTIBIOTICS, vol. 36, August 1983, pages 1040-1044; H. OGAWA et al.: "Cosynthesis and protoplast fusion by mutants of bialaphos (AMPBA) producing Streptomyces hygroscopicus"**

**TRENDS IN BIOTECHNOLOGY, vol. 1, no. 2, 1983, pages 42-48, Elsevier Science Publishers B.V., Amsterdam, NL; D.A. HOPWOOD et al.: "Cloning Streptomyces genes for antibiotic production"**

(73) Proprietor: **MEIJI SEIKA KABUSHIKI KAISHA**
**4-16 Kyobashi 2-chome**
**Chuo-ku, Tokyo-To(JP)**

(72) Inventor: **Murakami, Takeshi**
**1274-7, Nurumizu**
**Atsugi-Shi Kanagawa-Ken(JP)**
Inventor: **Imai, Satoshi**
**20-312, Dream Heights 1403, Matano-Cho**
**Totsuka-Ku**
**Yokohama-Shi Kanagawa-Shi**
**Kanagawa-Ken(JP)**
Inventor: **Anzai, Hiroyuki**
**5394, Izumi-Cho Totsuka-Ku**
**Yokohama-Shi Kanagawa-Shi**
**Kanagawa-Ken(JP)**
Inventor: **Satoh, Atsuyuki**
**23-107, Dream Heights 1403 Matano-Cho**
**Totsuka-Ku**
**Yokohama-Shi Kanagawa-Ken(JP)**
Inventor: **Nagaoka, Kozo**
**5-705 Ookurayama Haimu 891-2, Mamedo-Cho Kohoku-Ku**
**Yokohama-Shi Kanagawa-Ken(JP)**

GENE, vol. 38, no. 1/3, 1985, pages 215-226, Elsevier Science Publishers, Amsterdam, NL; M.J. BIBB et al.: "Cloning and analysis of the promoter region of the erythromycin resistance gene (ermE) of Streptomyces erythraeus"

NATURE, vol. 309, no. 5967, May-June 1984, pages 462-464, London, GB; F. MALPARTIDA et al.: "Molecular cloning of the whole biosynthetic pathway of a Streptomyces antibiotic and its expression in a heterologous host"

BIO/TECHNOLOGY, vol. 2, no. 1, January 1984, pages 63-72, New York, US; J.F. MARTIN et al.: "Cloning and expression of antibiotic production genes"

(74) Representative: **Dr. Elisabeth Jung Dr. Jürgen Schirdewahn Dipl.-Ing. Claus Gernhardt** **P.O. Box 40 14 68 Clemensstrasse 30** **W-8000 München 40(DE)**

## Description

Field of the Art

This invention relates to Bialaphos-producing genes. More particularly, the present invention relates to DNA strands comprising genes coding for the steps of the Bialaphos biosynthesis route of Bialaphos genes.

Actinomycetes are the most important fermenting microorganisms widely utilized in microorganism industries as microorganisms producing useful substances, typically antibiotics.

If the recombinant DNA technique can be utilized for breeding of actinomycetes, elucidation of the genetic properties thereof or improvement of productivity of antibiotics, it would be expected to have effects of extreme value in industries.

Prior Art

To the best of our knowledge, nothing has heretofore been reported on cloning as a cluster of antibiotic biosynthetic genes (secondary metabolic product biosynthetic genes) of actinomycetes, probably because of the difficulty in elucidating biosynthetic routes of antibiotic production, except on Actinorhodine disclosed in "Nature", 309, 462 (1984).

Meanwhile, the antibiotic produced by aerobic cultivation of a microorganism belonging to the genus Streptomyces, particularly Streptomyces hygroscopicus SF 1293 strain (Japanese Patent Publication No. 639/1976) has been known under the name of "SF 1293 substance" or "Bialaphos", and is useful as a fungicide for agriculture or horticulture (Japanese Patent Laid-Open Publication No.82028/1973) and a herbicide (Japanese Patent Laid-Open Publication No.23282/1984). Particularly, concerning herbicidal uses, a wide scope of uses may be expected for its specific feature of low pollutative effect as remnant.

## SUMMARY OF THE INVENTION

The present invention is intended to serve for improvement of productivity of the Bialaphos antibiotic which is a secondary metabolic product by cloning of a Bialaphos producing gene of Streptomyces hygroscopicus SF 1293 gene.

More specifically, the present invention provides a DNA strand comprising a gene coding for at least a part of the Bialaphos bioxynthesis route and being selected from the group consisting of the following DNA strands (1) through (6):

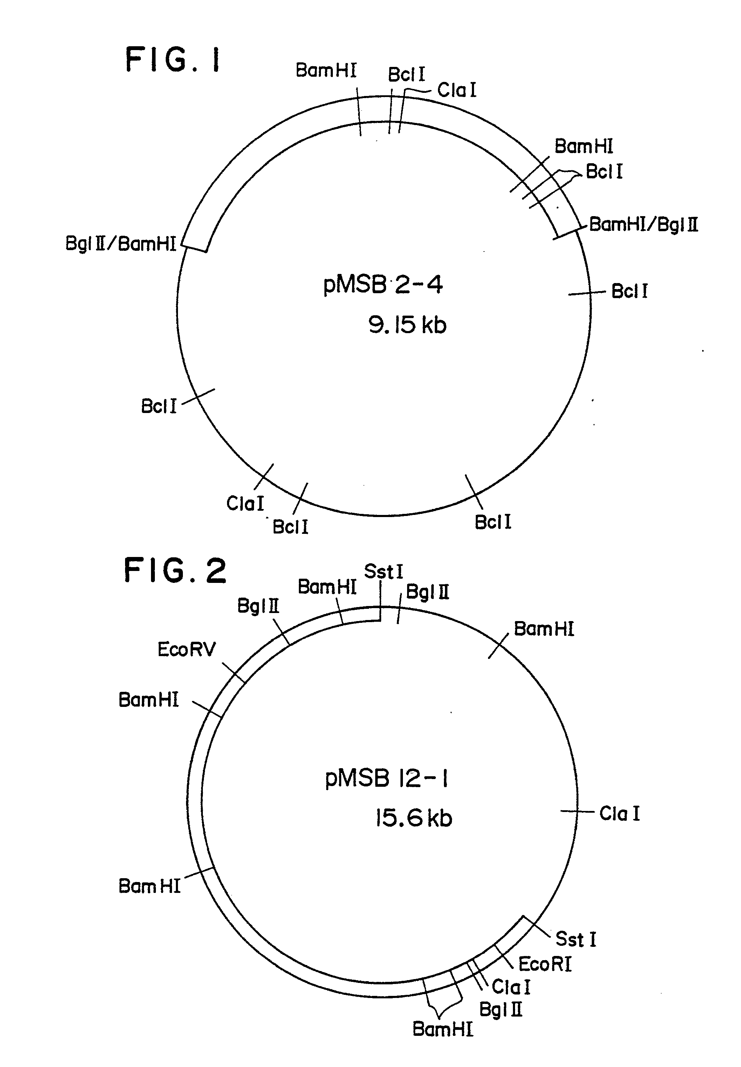

(1) a DNA strand equivalent to the DNA fragment derived from Bialaphos DNA in a hybrid plasmid pMSB 2-4 with a molecular weight of 9.15 kb having the restriction endonuclease cleavage site shown in Fig. 1, comprising a BamHI-digested fragment of Bialaphos DNA and a BglII-digested fragment of pIJ702 plasmid DNA;

(2) a DNA strand equivalent to the DNA fragment derived from Bialaphos DNA in a hybrid plasmid pMSB 12-1 with a molecular weight of 15.6 kb having the restriction endonuclease cleavage site shown in Fig. 2, comprising a SstI-digested fragment of Bialaphos DNA and a SstI-digested fragment of pIJ702 plasmid DNA;

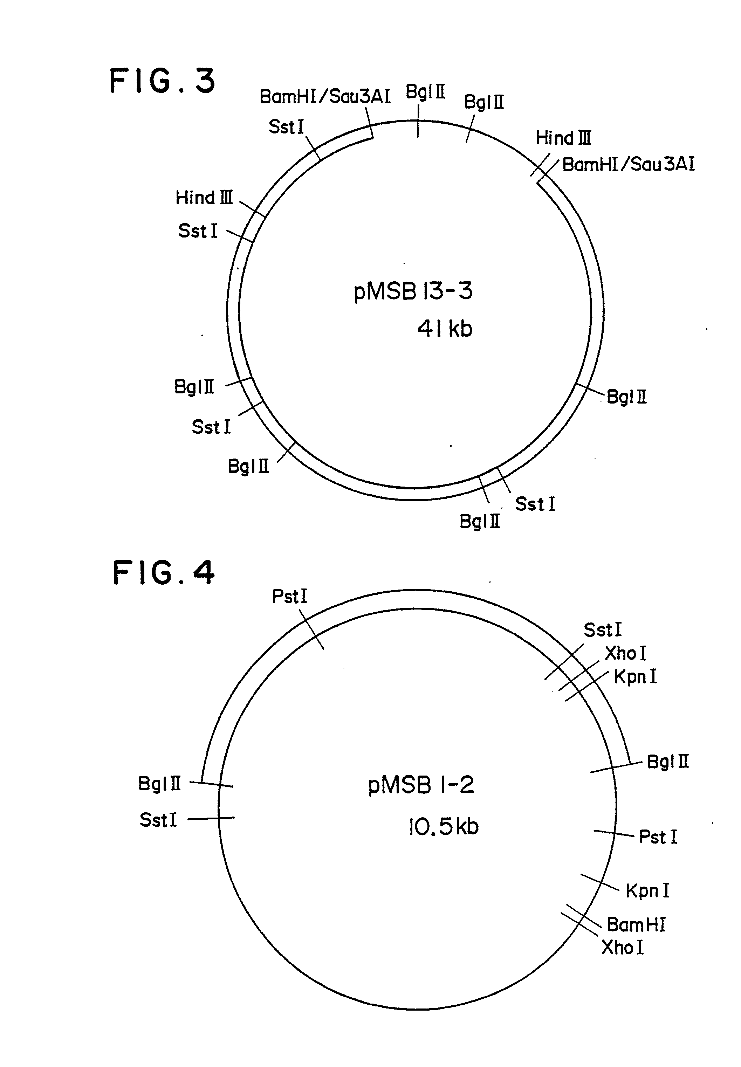

(3) a DNA strand equivalent to the DNA fragment derived from Bialaphos DNA in a hybrid plasmid pMSB 13-3 with a molecular weight of 41 kb having the restriction endonuclease cleavage site shown in Fig. 3, comprising a Sau3AI-digested fragment of Bialaphos DNA and a BamHI-digested fragment of cosmid pHC79 DNA;

(4) a DNA strand equivalent to the DNA fragment derived from Bialaphos DNA in a hybrid plasmid pMSB 1-2 with a molecular weight of 10.5 kb having the restriction endonuclease cleavage site shown in Fig. 4, comprising a BglII-digested fragment of Bialaphos DNA and a BglII-digested fragment of pIJ702 plasmid DNA;

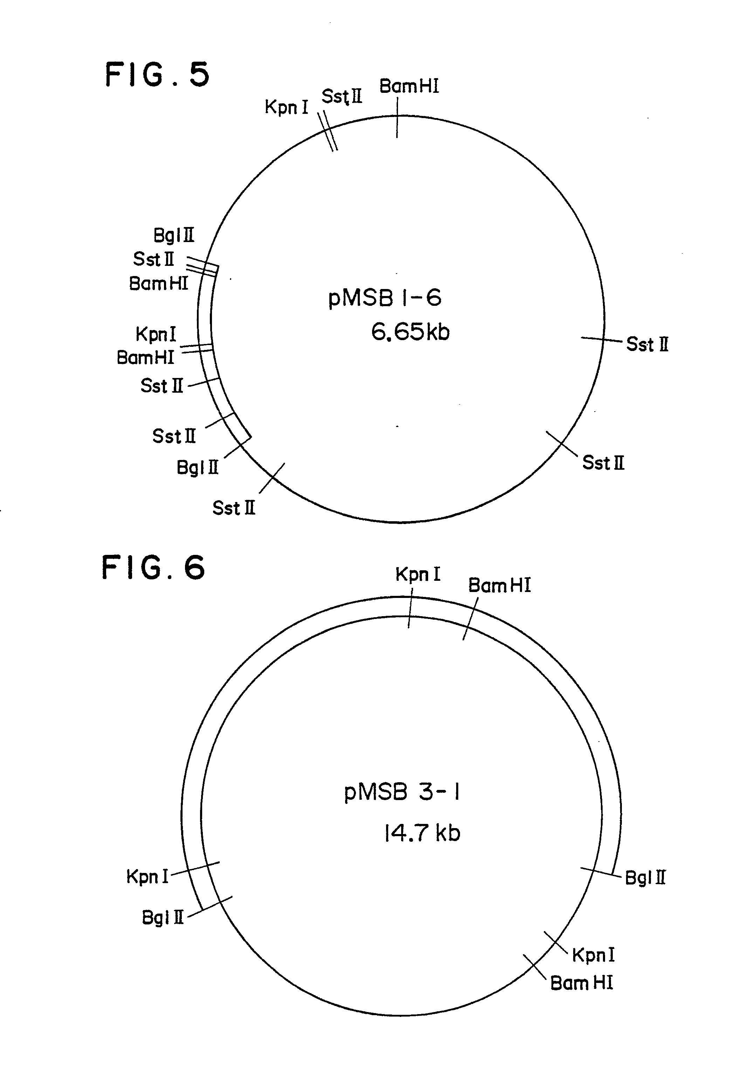

(5) a DNA strand equivalent to the DNA fragment derived from Bialaphos DNA in a hybrid plasmid pMSB 1-6 with a molecular weight of 6.65 kb having the restriction endonuclease cleavage site shown in Fig. 5, comprising a BglII-digested fragment of Bialaphos DNA and a BglII-digested fragment of pIJ702 plasmid DNA; and

(6) a DNA strand equivalent to the DNA fragment derived from Bialaphos DNA in a hybrid plasmid pMSB 3-1 with a molecular weight of 14.7 kb having the restriction endonuclease cleavage site shown in Fig. 6, comprising a BglII-digested fragment of Bialaphos DNA and a BglII-digested fragment of pIJ702 plasmid DNA.

Among these, the DNA strand (3) is most important.

The DNA fragments derived from Bialaphos DNA in the respective hybrid plasmids are thick portions of the DNA strands of the respective plasmids in Figs. 1 through 6.

By utilizing the DNA strands related to Bialaphos biosynthesis for the recombinant DNA technique, a contribution can be made to improvement of productivity of Bialaphos producing actinomycete.

## BRIEF DESCRIPTION OF THE DRAWINGS

In the drawings:

Figs. 1 through 6 are restriction endonuclease cleavage maps of plasmids pMSB 2-4, 12-1, 13-3, 1-2, 1-6 and 3-1, respectively;

Fig. 7 is a schematic diagram for illustration of the relationships between the respective DNA strands of the present invention incorporated in the plasmids pMSB 2-4, 12-1 and 13-3 and the Bialaphos biosynthetic gene; and

Fig. 8 is a schematic diagram for illustration of the Bialaphos biosynthetic route.

## DETAILED DESCRIPTION OF THE INVENTION

### DNA strands according to the present invention

Definition

The DNA strands according to the present invention are defined as follows.

In the present invention (also in the interpretation of the claim), the term "DNA strand" is inclusive, in addition to existence as a non-cyclic fragment, of the existence as a cyclic structure having said fragment incorporated therein, namely, a plasmid. A specific example of the latter case is a recombinant plasmid with pIJ 702 plasmid or pHC cosmid, typically pMSB13-3. Furthermore, the phrase "a DNA strand equivalent to the DNA fragment derived from Bialaphos DNA" in the hybrid plasmid is used to include DNA strands, in addition to the DNA strands (A) with the same base sequences as the DNA fragment derived from Bialaphos DNA, those (B) which have different base sequences due to degeneracy of DNA codons from but are the same in genotype as the DNA strands (A). The strand can be either a single strand or a double strand.

In the above description (and in Figs. 1 through 8), BamHI, etc. mean restriction endonucleases, as a matter of course.

Mutual relationships between DNA strands

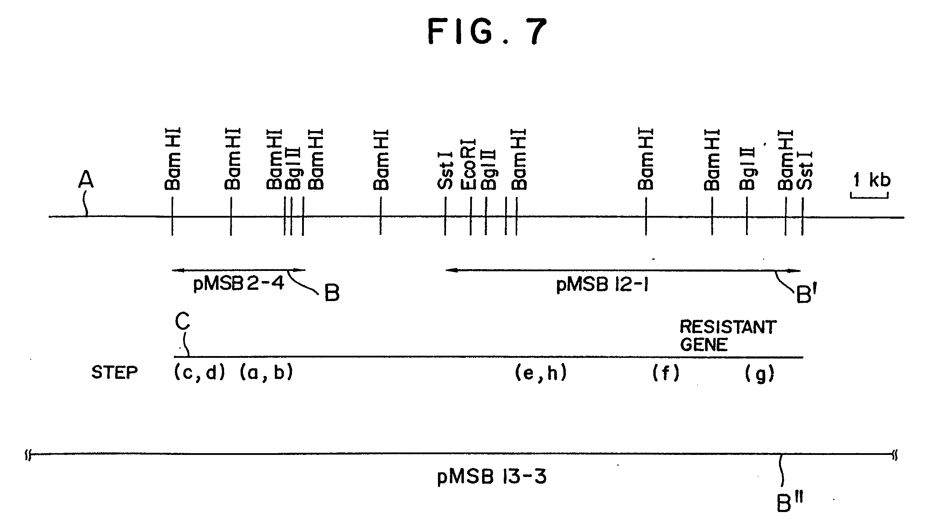

Fig. 7 shows to which sites of the Bialaphos biosynthetic gene, the DNA strands of the present invention in the respective hybrid plasmids of pMSB 2-4, 12-1 and 13-3 correspond; what their mutual relationships are; and at which sites the biosynthetic steps exist.

The straight line indicates the Bialaphos biosynthesis gene together with the restriction endonuclease site. The straight lines B, B' and B" are not pMSB 2-4, 12-1 and 13-1 themselves, but show the DNA strands of the present invention in the respective plasmids the straight line C shows the approximate positions of the genes coding for the steps a, b, c, d, e, f, g, and h of the biosynthetic route, respectively.

Bialaphos/SF 1293 substance

Chemical entity

The antibiotic produced by Streptomyces hygroscopicus SF 1293 microorganism (FERM BP-130, ATCC 21705) has been known as Bialaphos or SF 1293 substance, as mentioned above.

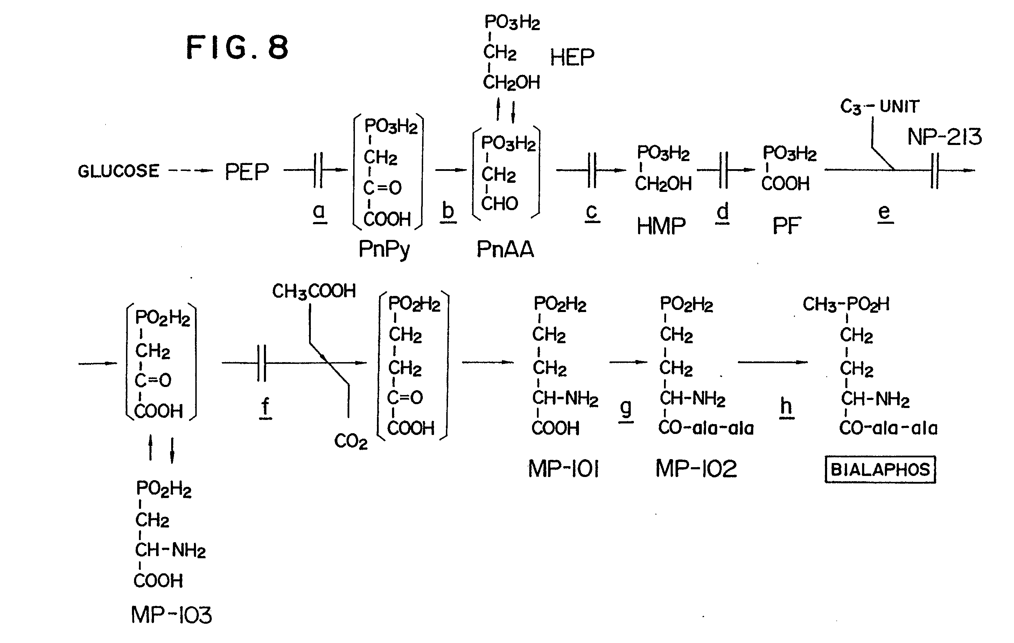

The molecular formula of Bialaphos is as shown in Fig. 8.

This antibiotic may herein be called as Bialaphos or as SF 1293 substance, which should be understood as being identical.

Biosynthesis route

The biosynthetic route of Bialaphos is as shown in Fig. 8 (now being contributed to a journal J. Antibiotic).

In this biosynthesis route, a through h are unit steps for biosynthesis, but each step does not necessarily mean one step reaction from the starting substance to the product substance (for example, in step a, PEP and PnPY) but also includes the case comprising multi-step reactions.

The microorganism related to the present invention are on deposit for those required (see in detail the item of "Deposition of related microorganisms" given below).

## Cloning of Bialaphos-producing gene

### Cloning

In the cloning of a Bialaphos-producing gene, Bialaphos-producing gene DNA was extracted and purified according to the conventional method, namely, the method of Smith et al. (Method in Enzymology, 12, 545, 1967), from Streptomyces hygroscopicus SF 1293 and mutant strains derived therefrom, and this DNA was combined with a vector plasmid pIJ 702 plasmid (available from John Innes Research Institute; J. General Microbiology, 129, 2703 - 2714, 1983) to form a hybrid plasmid. With the use of this hybrid plasmid, biosynthesis deficient strains (strains deficient in genes coding for at least one of the steps a through h of the biosynthesis route as mentioned above) were transformed, and the presence or absence of restoration of Bialaphos production in the deficient strains was examined.

More specifically, two different types of biosynthesis strain (Strain NP-50 deficient in step a and Strain NP-52 deficient in step f) were used as hosts, respectively, and the DNA fragment for restoring production ability was put into pIJ 702 as the vector by shot-gun cloning. The plasmid pMSB 2-4 (9.15 kilobase (kb), deposited with NP-50 strain as the host; FERM P-7805) was found to restore production of Bialaphos not only in the host strain but widely in other biosynthesis deficient strains. As a result of subcloning, the plasmid pMSB 2-4 was found to contain four kinds of genes participating in the initial reactions of biosynthesis (genes concerning steps a, b, c, and d), and pMSB 12-1 to contain four kinds of genes of the latter half of biosynthesis (genes concerning steps e, f, g, and h) (FERM P-7807). Further, Escherichia coli LE 392 (FERM P-7477) was used to prepare a cosmid library of DNA of Streptomyces hygroscopicus SF-1293 strain, to which pMSB 2-4 and pMSB 12-1 were hybridized as probe, respectively. As a result, both probes were found to be hybridized with the same cosmid pMSB 13-3. It may be considered that most of the biosynthetic genes of the herbicidal active substance Bialaphos biosynthesized in ten and several steps from phosphoenol pyruvic acid can be cloned on pMSB 13-3. The cosmid 13-3 (41 kb) is on deposit with E. coli LE 392 as the host (FERM P-7808).

Similar experiments were conducted with the use of Streptomyces hygroscopicus 369-IS strain (FERM P-7809) as the host, the plasmid pMSB 1-2 (10.5 kb; on deposit with 369-IS strain as the host; FERM P-7810) and pMSB 1-6 (6.65 kb; on deposit with 369-IS strain as the host; FERM P-7811). The 369-IS strain is not a biosynthesis deficinet strain but is a strain of low Bialaphos producing ability. Bialaphos productivity of the 369-IS strain is improved by transformation thereof with pMSB 1-2. On the other hand, in the case of transformation thereof with pMSB 1-6, activity of the producing strains is observed to be suppressed.

Also, similar experiments were conducted for the biosynthesis deficient strain NP-51 (FERM P-7812) with the use of a plasmid pMSB 3-1 (14.7 kb; on deposit with NP51 strain as the host; FERM P-7813). Although the NP51 strain is a biosynthesis deficient strain, it is not clear as to which step of the biosynthetic route is deficient, but restoration of Bialaphos productivity in NP51 by transformation was confirmed.

The above experiments were carried out according to the methods conventionally used for recombination of genes, and the details are shown in the experimental examples set forth hereinafter. From these, it would be possible for those skilled in the art to practice the present invention.

### Deposition of related microorganisms

The microorganisms related to the present invention have been deposited at the Fermentation Research Institute, Agency of Industrial Science and Technology, Ministry of International Trade and Industry, 1-3, Higashi I-chome, Yatabe-machi, Tsukuba-gun, Ibaraki-ken, 305, Japan.

| *1, *3 Microorganism | *2 Deposit Number | Deposition Date |
|---|---|---|
| SF 1293 | BP-130 (ATCC 21705) | Jun. 25, 1971 |
| NP50 | P-7804 | Aug. 24, 1984 |
| NP50 (pMSB 2-4) | p-7805 | Aug. 24, 1984 |
| NP44 | P-7806 | Aug. 24, 1984 |
| NP44 (pMSB 12-1) | p-7807 | Aug. 24, 1984 |
| LE 392 | P-7477 | Feb. 28, 1984 |
| LE 392 (pMSB 13-3) | P-7808/ BP-853 | Aug. 24, 1984 |
| 369-1S | P-7809 | Aug. 24, 1984 |
| 369-1S (pMSB 1-2) | P-7810 | Aug. 24, 1984 |
| 369-1S (pMSB 1-6) | P-7811 | Aug. 24, 1984 |
| NP51 | P-7812 | Aug. 24, 1984 |
| NP51 (pMSB 3-1) | P-7813 | Aug. 24, 1984 |

*1) All are Streptomyces hygroscopicus except for LE 392 which is Escherichia coli.

*2) P- shows "FERM P-" and BP shows "FERM BP-", respectively.

*3) (1) The bacteriological properties of NP microorganisms and 369-1S microorganisms are the same as those of the Streptomyces hygroscopicus SF 1293 strain (FERM BP-130) (ATCC 21705) which is the parent strain thereof, except for the genetic trait of Bialaphos biosynthesis deficiency, as disclosed in Japanese Patent Publication No. 639/1976.

(2) The bacteriological properties of Escherichia coli LE 392 are disclosed in "Molecular Cloning" (edited by T. Maniatis, Cold Spring Harbor Laboratory).

Streptomyces lividans 66 (FERM P-6982) has also been deposited.

The above LE 392 (pMSB 13-3) FERM P-7808 now stands as an international deposit under BUDAPEST TREATY ON THE INTERNATIONAL RECOGNITION OF THE DEPOSIT OF MICROORGANISMS FOR THE PURPOSE OF PATENT PROCEDURE, and satisfies the requirements of said TREATY and is releasable under the provisions of said TREATY.

Experimental Examples

The SF 1293 biosynthetic deficient strains comprise those under the deficient states as shown below:

| Deficient strain (Streptomyces hygroscopicus) | Deficient step |
|---|---|
| NP50 | a |
| NP47 | a |
| NP33 | a and b |
| NP46 | c |
| NP221 | d |
| NP213 | e |
| NP52 | f |
| NP44 | f |
| NP45 | g |
| NP8 | g and h |

Example 1 (Preparation of pMSB 2-4)

Streptomyces hygroscopicus SF-1293 was inoculated into 10 ml of S-I medium (2% soluble starch, 1.0% peptone, 0.3% meat extract, 0.05% $K_2HPO_4$, pH 7.0), cultured at 28° C for 24 - 48 hours, and the resultant culture broth was used as the seed microorganism and subinoculated into a MYG medium at a concentration of inoculated microorganism of 5%. The MYG medium was prepared by adding 2% glycine to 1% malt extract, 0.4% yeast extract, 0.5% glucose, pH 7.0 (80 ml). This was subjected to shaking cultivation at 28° C for 24 hours, and the microorganism was collected by centrifugation at 10,000xg/10 min.

From the microorganism cells, all DNA were extracted and purified according to the known method of Smith et al. (Methods in Enzymology 12, 545, 1967), and dialyzed against a TESC buffer (10 mM Tris-HCl, pH 8.0, 1 mM EDTA, 2.5 mM NaCl) to produce a donor DNA.

The thus obtained donor DNA (10 μg) was incubated at 37° C for 2 hours with addition of 10 units of a restriction endonuclease BamHI in a reaction medium of 200 μl total volume with a composition of 20 mM Tris-HCl, pH 7.4, 50 mM NaCl, 10 mM $MgCl_2$ and 10 mM β-meraptoethanol.

The plasmid pIJ702 DNA (2 μg) was digested at 37° C for 2 hours with 2 units of a restriction endonuclease BglII in a reaction medium of 40 μl total volume with a composition of 20 mM Tris-HCl, pH 7.4, 10 mM $MgCl_2$ and 10 mM β-meraptoethanol.

The reaction mixture was heated at 70° C for 10 minutes to deactivate the enzyme, then, with addition of 1/10 vol. 500 mM NaCl and 10 units of calf intestine alkaline phosphatase (c.i.a.p.), digestion was carried out at 37° C for 60 minutes.

To each of the reaction mixtures of the donor DNA and the plasmid DNA was added an equal volume of a phenol solution saturated with a TESH buffer (0.2 M Tris-HCl, pH 8.0, 20 mM dithiothreitol, 50 mM NaCl), which step was followed by shaking for one minute, and the two mixtures were combined. After the combined mixture was centrifuged at 10,000xg/5 min., the upper layer (aqueous layer) was taken out with a Pasteur pipette.

The aqueous layer portion containing DNA was subjected to ethyl ether extraction to remove phenol, and an equal volume of ethanol was added. After the mixture was left to stand at -80° C for 2 hours, DNA was precipitated by centrifugation at 10,000xg/5 min.

The DNA thus obtained was washed with 500 μl of ethanol, dried under reduced pressure, and dissolved in 50 μl of heat-sterilized pure water. The DNA was subjected to heat treatment at 70° C for 10 minutes, then cooled gradually to room temperature and inoculated with addition of 5 μl of a buffer (0.66 M Tris-HCl, pH 7.6, 66 mM $MgCl_2$, 0.1 M dithiothreitol, 10 mM ATP) and 5 μl (1 unit) of T4 ligase at 22° C for 2 hours to prepare a recombinant DNA of pIJ702 and the Streptomyces hygroscopicus DNA.

Transformation of the host microorganism Streptomyces hygroscopicus NP50 with this DNA was carried out according to the protoplast PEG method conventionally used for actinomycetes (e.g. Curr. Topics Microbiol. Immunol., 96, 69, 1981).

More specifically, Streptomyces hygroscopicus NP50 was inoculated into 80 ml of S medium (glycose 1%, peptone 0.4%, yeast extract 0.4%, $MgSO_4$ $7H_2O$ 0.05%, $KH_2PO_4$ 0.2%, $K_2HPO_4$ 0.4%, glycine 5%), and shaking culture was carried out at 28° C for 18 hours. Mycelia were collected by centrifugation at 10,000xg, washed once with 0.5 M sucrose solution, and then suspended in 10 ml of Ps medium (NaCl 70 mM, sucrose 0.5 M, MgCl₂ 5 mM, CaCl₂ 5 mM, 25 mM TES pH 7.2). Then egg white lysozyme and Achromo-peptidase were added to obtain a final concentration of 1 mg/ml and 0.5 mg/ml, respectively, and incubation was then carried out at 37°C for 60 minutes to form protoplasts. The mycelia not formed into protoplasts were removed by cotton filtration. The protoplasts were collected by centrifugation at 800xg/10 min., washed once with Ps medium, and suspended in 2 ml of Ps medium.

A mixture of 100 μl of the protoplast suspension, 100 μl of a P-maleic acid buffer adjusted to 3/2 concentration (2.5% sucrose, 1.4 mM K₂SO₄, 1/500 vol. trace elements solution, 100 mM CaCl₂, 50 ml Tris-maleate pH 8.0), 50 μl of the recombinant DNA solution and 375 μl of a polyethylene glycol solution (polyethylene glycol #1000, 33%/P-maleate buffer) was left to stand at room temperature for one minute then diluted with 5 ml of Ps medium, and the protoplasts were collected by centrifugation at 800xg/10 min. and suspended in 2 ml of Ps medium.

After aliquots of the protoplast suspension each of 0.1 ml were applied onto RH agar media prepared in 20 circular plastic petri dishes of 9 cm in diameter (containing, in one liter, sucrose 171 g, KCl 14.9 g, glucose 10 g, trace element solution 2 ml, 2.5% K₂SO₄ 10 ml, proline 3 g, casamino acid (CAS) 0.5 g, polypeptone 2 g, dextran 0.05 g, 0.5% KH₂PO₄ 10 ml, 1M CaCl₂ 2H₂O 50 ml, 0.25M TES (pH 7.2) 100 ml, CSL 30g/1000 ml 100 ml, 5% aspartic acid 10 ml), 2 ml of RH medium with an agar concentration of 0.5% was overlaid as the upper layer. On this layer, after cultivation at 28°C for 18 hours, was further overlaid 2 ml of the same RH (soft agar 0.5%) to which thiostrepton had been added to 20 μg/ml. This was continued to be cultured at 28°C, in a chamber maintained at a humidity of 90%, for 10 to 20 days. The microorganism on the RH agar medium regenerated from protoplasts was transferred onto a nutrient agar medium containing 1% CAS, 5 μg/ml CuSO₄·5H₂O and 0.5 mg/ml thyrosine by means of a commercially available needle. This was subjected to culturing at 28°C for 2 days. As the result, colonies producing melanine dyes and colonies producing no melanine dye were formed in a ratio of about 1:1. Incidentally, "CSL" means corn steep liquor.

Since Streptomyces hygroscopicus DNA is incorporated at the BglII site on the gene participating in the production of melanine of the vector pIJ702, the clone introduced by the plasmid having foreign Streptomyces hygroscopicus DNA incorporated therein upon ligation will not produce melanine. However, the plasmid having no Streptomyces hygroscopicus DNA incorporated at BglII site produces melanine dyes. Thus, the colonies which do not produce melanine dyes can be judged to have Streptomyces hygroscopicus DNA incorporated on the plasmid introduced.

Of these, the colonies producing no melamine dye was replicated onto an agar piece having a composition as shown below by the use of a commercially available tooth-pick. The agar piece was a column of agar of 7 to 8 mm in diameter and 4 - 5 mm in height punched out with a cork borer from a composition comprising 0.4% glucose, 0.35% wheat germ, 0.23% SVP (soluble vegetable protein), 0.03% KH₂PO₄, 0.0001% CoCl₂·6H₂O, 0.2% agar, pH 7.0.

The agar piece was subjected to cultivation at 28°C for 6 days. This was examined as to whether or not it was an agar piece forming an inhibitory circle at 32°C with the use of Proteus sp. MB-838 as the test microorganism. As a result, of 2500 colonies, 5 strains of colonies restored in Bialaphos production were obtained.

The 5 strains of Streptomyces hygroscopicus which are thiostrepton resistant and yet restored in Bialaphos production capacity were subjected to seed culturing similarly as described above and inoculated, each in a quantity of 80 ml, into MYG medium (to which 2% glycine had been added). From the microbial cells after shaking culturing at 28°C for 24 hours, the plasmid was extracted according to the known method by Hansen et al. (J. Bacteriol. 135, 227, 1978). When Streptomyces hygroscopicus NP50 and its deficient mutant Streptomyces hygroscopicus NP47 with the same biosynthetic route were again transformed with these plasmids, every transformant could be restored in production of Bialaphos.

As to whether or not these microorganism strains produced Bialaphos, analysis was performed according to the method as disclosed in a Literature "Annual Research Report by Meiji Seika No.21, 44 - 51, 1982". Briefly, the filtrate of each microorganism strain culture broth was analyzed by an amino acid analyzer, and the antimicrobial substance produced in the medium could be identified chromatographically as Bialaphos.

Also, the plasmid pMSB 2-3, 2-4, 2-5, 2-7 and 2-8 obtained from these 5 strains were cleaved with a restriction endonuclease BamHI, and the fragments obtained were examined by 1% agarose electrophoresis, whereupon it was found that all of them contained commonly the BamHI fragment of 1.32 kb. Also, since the plasmid in which the minimum DNA fragment is encoded is pMSB 2-7, the enzyme gene for performing the reaction of the step a in Bialaphos biosynthesis is encoded in the BamHI fragment of 1.32 kb.

Among these, for examination as to whether other genes are encoded in the plasmid pMSB 2-4 with the greatest inserted fragment, pMSB 2-4 was inserted into the Bialaphos biosynthesis deficient strains NP33, NP46 and NP221. As a result, any one of the deficient strains could be restored in Bialaphos productivity.

This result shows that the pMEB 2-4 codes for the enzyme genes for the steps a - d participating in Bialaphos biosynthesis.

Example 2 (Preparation of pMSB 12-1)

Streptomyces hygroscopicus SF-1293 was inoculated into 10 ml of S-I medium (2% soluble starch, 1.0% peptone, 0.3% meat extract, 0.05% $K_2HPO_4$, pH 7.0), cultured at 28°C for 24 - 48 hours, and the resultant culture broth was used as the seed microorganism and subinoculated into an MYG medium at a concentration of inoculated microorganism of 5%. The MYG medium was prepared by adding 2% glycine to 1% malt extract, 0.4% yeast extract, 0.5% glucose, pH 7.0 (80 ml). This was subjected to shaking cultivation at 28°C for 24 hours, and the microorganism was collected by centrifugation at 10,000xg/10 min. From the microorganism cells, all DNA were extracted and purified according to the known method of Smith et al. (Methods in Enzymology 12, 545, 1967), and dialyzed against a TESC buffer (10 mM Tris-HCl, pH 8.0, 1 mM EDTA, 2.5 mM NaCl) to obtain a donor DNA.

The donor DNA (10 µg) thus obtained was digested at 37°C for 2 hours with 10 units of a restriction endonuclease SstI in a reaction medium of 200 µl total volume with a composition of 20 mM Tris-HCl, pH 7.4, 50 mM NaCl, 10 mM $MgCl_2$ and 10 mM $\beta$-meraptoethanol.

The plasmid pIJ702 DNA (2 µg) was digested at 37°C for 2 hours with 2 units of a restriction endonuclease SstI in a reaction medium of 40 µl total volume with a composition of 20 mM Tris-HCl, pH 7.4, 10 mM $MgCl_2$ and 10 mM $\beta$-meraptoethanol.

The reaction mixture was heated at 70°C for 10 minutes to deactivate the enzyme, then, with addition of 1/10 vol. 500 mM NaCl and 10 units of calf intestine alkaline phosphatase (c.i.a.p.), incubation was carried out at 37°C for 60 minutes.

To each of the reaction mixtures of the donor DNA and the plasmid DNA was added an equal volume of a phenol solution saturated with a TESH buffer (0.2 M Tris-HCl, pH 8.0, 20 mM dithiothreitol, 50 mM NaCl), and thereafter shaking was carried out for one minute, and both of the mixtures were combined. After the combined mixture was centrifuged at 10,000xg/5 min., the upper layer (aqueous layer) was taken out with a Pasteur pipette.

The aqueous layer portion containing DNA was subjected to ethyl ether extraction to remove phenol, and an equal volume of ethanol was added. After the mixture was left to stand at -80°C for 2 hours, DNA was precipitated by centrifugation at 10,000xg/5 min.

The DNA thus obtained was washed with 500 µl of ethanol, dried under reduced pressure and dissolved in 50 µl of heat-sterilized pure water. The DNA was subjected to heat treatment at 70°C for 10 minutes, then cooled gradually to room temperature and incubated with addition of 5 µl of a buffer (0.66 M Tris-HCl, pH 7.6, 66 mM $MgCl_2$, 0.1 M dithiothreitol, 10 mM ATP) and 5 µl (1 unit) of T4 ligase at 22°C for 2 hours to prepare a recombinant DNA of pIJ702 and the Streptomyces hygroscopicus DNA.

Transformation of the host microorganism Streptomyces hygroscopicus NP52 with this DNA was accomplished according to the protoplast PEG method conventionally used for actinomycetes (e.g. Curr. Topics Microbiol. Immunol., 96, 69, 1981).

More specifically, Streptomyces hygroscopicus NP52 was inoculated into 80 ml of S medium (glucose 1%, peptone 0.4%, yeast extract 0.4%, $MgSO_4 \cdot 7H_2O$ 0.05%, $KH_2PO_4$ 0.2%, $K_2HPO_4$ 0.4%, glycine 5%); shaking culturing was carried out at 28°C for 18 hours; and mycelia were collected by centrifugation at 10,000xg, washed once with 0.5 M sucrose solution, and then suspended in 10 ml of Ps medium (NaCl 70 mM, sucrose 0.5 M, $MgCl_2$ 5 mM, $CaCl_2$ 5 mM, 25 mM TES pH 7.2). Then egg white lysozyme and Achromo-peptidase were added to obtain a final concentration of 1 mg/ml and 0.5 mg/ml, respectively, which step was followed by incubation at 37°C for 60 minutes to form protoplasts, and the mycelia not formed into protoplasts were removed by cotton filtration. The protoplasts were collected by centrifugation at 800xg/10 min., washed once with Ps medium, and suspended in 2 ml of Ps medium.

A mixture of 100 µl of the protoplast suspension, 100 µl of a P-maleate buffer adjusted to 3/2 concentration (2.5% sucrose, 1.4 mM $K_2SO_4$, 1/500 vol. trace elements solution, 100 mM $CaCl_2$, 50 ml Tris-maleate, pH 8.0), 50 µl of the recombinant DNA solution and 375 µl of a polyethylene glycol solution (polyethylene glycol #1000, 33 %/P-maleate buffer) was left to stand at room temperature for one minute then diluted with 5 ml of Ps medium, and the protoplasts were collected by centrifugation at 800xg/10 min. and suspended in 2 ml of Ps medium.

After aliquots of the protoplast suspension each of 0.1 ml were coated onto RH agar media prepared in

EP 0 173 327 B1

20 circular plastic petri dishes of 90 cm in diameter (containing, in one liter, sucrose 171 g, KCl 14.9 g, glucose 10 g, trace element solution 2 ml, 2.5% $K_2SO_4$ 10 ml, proline 3 g, casamino acid 0.5 g, polypeptone 2 g, dextran 0.05 g, 0.5% $KH_2PO_4$ 10 ml, 1M $CaCl_2 \cdot 2H_2O$ 50 ml, 0.25M TES (pH 7.2) 100 ml, CSC 30g/1,000 ml 100 ml, 5% aspartic acid 10 ml), 2 ml of RH medium with an agar concentration of 0.5% was overlaid as the upper layer. On this layer, after cultivation at 28°C for 18 hours, was further overlaid 2 ml of the same RH (soft agar 0.5%) with the addition of thiostrepton to 20 μg/ml. This was continued to be cultured at 28°C in a chamber maintained at a humidity of 90% for 10 to 20 days. The microorganism on the RH agar medium regenerated from protoplasts was transferred onto a nutrient agar medium containing 1% CAS, 5 μg/ml $CuSO_4 \cdot 5H_2O$ and 0.5 mg/ml thyrosine by means of a commercially available needle. This was subjected to culturing at 28°C for 2 days. As a result, colonies producing melanine dyes and colonies producing no melanine dye were formed in a ratio of about 1:1.

Since Streptomyces hygroscopicus DNA is incorporated at the SstI site on the gene participating in the production of melanine of the vector pIJ702, the clone introduced by the plasmid having foreign Streptomyces hygroscopicus DNA incorporated therein upon ligation will not produce melanine. However, the plasmid having therein no Streptomyces hygroscopicus DNA incorporated at the SstI site produces melanine dyes. Thus, the colonies which do not produce melanine dyes can be judged to have Strentomyces hygroscopicus DNA incorporated on the plasmid introduced.

Of these, the colonies producing no melanine dye were replicated onto the agar piece having a composition as shown below by the use of a commercially available tooth-pick. The agar piece is a column of agar of 7 to 8 mm in diameter and 4 - 5 mm in height punched out with a cork borer from a composition comprising 0.4% glucose, 0.35% wheat germ, 0.23% SVP (soluble vegetable protein), 0.03% $KH_2PO_4$, 0.0001% $CoCl_2 \cdot 6H_2O$, 0.2% agar, pH 7.0.

The agar piece was subjected to cultivation at 28°C for 6 days. This was examined as to whether or not it was an agar piece forming an inhibitory circle at 32°C with the use of Proteus sp. MB-838 as the test microorganism. As a result, of 1,000 colonies, one strain of colony restored in production was obtained.

The one strain of Streptomyces hygroscopicus which is thiostrepton resistant and yet is restored in Bialaphos production capacity was subjected to seed culturing similarly as described above and inoculated, in quantities each of 80 ml, into MYG medium (added with 2% glycine). From the microorganism cells after shaking culturing at 28°C for 24 hours, the plasmid was extracted according to the known method by Hansen et al. (J. Bacteriol. 135, 227, 1978). When deficient mutants Streptomyces hygroscopicus NP52 and Streptomyces hygroscopicus NP44 with the same biosynthetic route were again transformed with these plasmids. Every transformant could be restored in the production of Bialaphos.

As to whether or not these microorganism strains produced Bialaphos, analysis was performed according to the method as disclosed in "Annual Research Report by Meiji Seika No. 21, 44-51, 1982". Briefly, the filtrate of each microorganism strain culture broth was analyzed with an amino acid analyzer, and the antimicrobial substance produced in the medium could be identified chromatographically as Bialaphos.

Further, the plasmid obtained from this strain was cleaved with a restriction endonuclease SstI, and the fragments obtained were examined by 1% agarose electrophoresis (according to the method and the conditions generally practiced) to find that a DNA fragment derived from Streptomyces hygroscopicus of 9 kb was contained at the SstI site of pIJ702.

Next, since pMSB 12-1 was also estimated to contain genes participating in respective steps of biosynthesis other than the genes restoring productivity in the respective biosynthesis deficient strains of NP52 and NP44, pMSB 12-1 was introduced into other biosynthesis deficient strains NP213, NP8 and NP45, respectively. The method for introduction was the same as the transforming method as described above. As a result, it has become clear that the transformants can also restore the Bialaphos producing ability.

Next, pMSB 12-1 was introduced into Streptomyces lividans 66 (deposited as FERM P-6982 at Fermentation Research Institute, Agency of Industrial Science & Technology, on March 10, 1983, the bacteriological properties of which are described in Japanese Patent Application No. 52277/1983). The method is similar to that disclosed in Curr. Topics Microbiol. Immunol., 96, 69, 1981.

For examination of the properties of Streptomyces lividans 66 having pMSB 12-1 introduced thereinto, each of S. lividans 66 containing pMSB 12-1 and S. lividans 66 containing no pMSB was smeared on R2YE medium to be cultivated thereon to form spores. The spores were smeared on the medium prepared by controlling previously Bialaphos to a 1,000 μg/ml concentration on a commercially available nutrient agar produced by Difco Co., and cultivation was carried out at 37°C for 3 days. As a result, S. lividans containing pMSB 12-1 was found to grow, but S. lividans of the wild strains could not grow. The wild strain can grow in a medium of 200 μg/ml under the same conditions.

Thus, it has been made clear that the S. lividans having pMSB 12-1 introduced therein is improved in resistance to Bialaphos by 2-fold to 5-fold as compared with the strain containing no pMSB 12-1. This means that pMSB 12-1 contains a resistant gene against Bialaphos.

Thus, it can be seen that pMSB 12-1 codes for not only the respective genes shown below, but also codes for a resistant gene against Bialaphos.

Gene for restoring producing ability of NP52 (Step f)
Gene for restoring producing ability of NP44 (Step f)
Gene for restoring producing ability of NP8 (Steps f and h)
Gene for restoring producing ability of NP45 (Step g)
Gene for restoring producing ability of NP213 (Step e)

Example 3 (Preparation of pMSB 13-3)

Streptomyces hygroscopicus SF-1293 was inoculated into 10 ml of S-I medium (soluble starch 2%, peptone 1.0%, meat extract 0.3%, $K_2HPO_4$ 0.05%, pH 7.0) and subjected to shaking cultivation at 28°C for 2 days, and 4 ml of the pre-culture thus obtained was inoculated into 80 ml of a MYG medium + glycine medium (malt extract 1%, yeast extract 0.4%, glucose 2%, glycine 2%, pH 7.0), which step was followed by shaking cultivation at 28°C overnight. The culture broth was subjected to centrifugation at 10,000xg/10 min. to collect microorganisms. From the microorganism cells, all DNA were extracted and purified according to the known method of Smith et al. (M.G. Smith: Methods in Enzymology 12, 545, 1967), and dialyzed against a TE buffer (10 mM Tris-HCl, pH 8.0, 1 mM EDTA) to obtain a donor DNA.

The thus obtained donor DNA (10 mg) was digested for 10 minutes with 2 units of a restriction endonuclease Sau3A in 50 μl of a buffer with a composition of 10 mM Tris-HCl, pH 7.2, 10 mM dithiothreitol, 150 mM NaCl. To the reaction mixture was added 50 μl of a phenol solution saturated with the TE buffer, after which shaking was carried out for one minute, and the mixture was centrifuged at 10,000xg/5 min., the upper layer (aqueous layer) was taken out with a Pasteur pipette. The aqueous layer containing DNA was extracted twice with ethyl ether to remove phenol, and then DNA was precipitated by centrifugation at 10,000xg/5 min. The DNA thus recovered was washed once with 500 μl of ethanol, dried under reduced pressure, and dissolved in 20 μl of pure water sterilized by heating to provide a donor DNA.

On one hand, 2 μg of a cosmid vector pHC 79 (Barbra Hohn et al.: Gene, 11, 291-298, 1980; commercial product) was digested with 4 units of a restriction endonuclease BamHI at 37°C for 2 hours in 20 μl of a buffer with a composition of 10 mM Tris-HCl, pH 7.2, 10 mM dithiothreitol, 100 mM NaCl. Further, after heating at 75°C for 10 minutes, one unit of a commercially available alkaline phosphatase derived from E. coli was added to carry out incubation at 50°C for one hour. Then, following phenol treatment, ethanol precipitation and ethanol washing, the reaction product was dissolved as the vector DNA in 10 μl of sterilized water.

A mixture of 20 μl of the above donor vector and 10 μl of the above vector DNA was subjected to heat treatment at 70°C for 10 minutes, then cooled gradually to room temperature, and incubated with addition of 4 μl of a buffer (0.66 M Tris-HCl, pH 7.6, 66 mM $MgCl_2$, 0.1 M dithiothreitol, 10 mM ATP) and 2 μl of T4 DNA ligase at 22°C for 14 hours to prepare a recombinant DNA of pHC 79 and the Streptomyces hygroscopicus DNA.

Transformation of the host microorganism E. coli LE392 with this recombinant DNA was carried out according to the known method of Horn et al. (methods in Enzymology, 68, 299 - 309, 1979). More specifically, 10 μl of ethanol was added to 36 μl of the previously prepared recombinant DNA of pHC 79 and Streptomyces hygroscopicus DNA, and the mixture was left standing at -80°C for 2 hours and then subjected to centrifugation at 10,000xg/5 min. to recover the recombinant DNA as the precipitate. The precipitate was dissolved with addition of 20 μl of heat sterilized water, and 10 μl of the solution was added into 50 μl of a phage particle forming solution in a test tube which was prepared according to the method of Hohn et al. and commercially sold to carry out incubation at 37°C for one hour. This step was further followed by incubation at 37°C for 10 minutes with addition of 150 μl of a buffer with a composition of 10 mg/μl DNase I, 10 mM Tris-HCl, pH 7.4, 10 mM $MgCl_2$, 0.1 M NaCl, 0.03% BSA. Thereafter, the solution was subjected to centrifugation at 10,000xg/5 min. to remove the supernatant to cause the recombinant to become incorporated within the lambda-phage particles.

Transduction into E. coli K-12 LE392 was conducted as follows. 2 ml of LE392 was inoculated into L medium containing 0.1% maltose (1% polypeptone, 0.5% yeast extract, 0.5% NaCl), and, after cultivation overnight, the phage particle solution with previously introduced recombinant DNA was added, and aliquots, each of 0.1 ml of the mixture, were applied onto laboratory dishes containing L medium containing 1.5% agar, 50 μg/ml ampicillin. Cultivation was conducted at 37°C overnight to form colonies, thus causing LE392 having the recombinant DNA to appear as colonies. Selection of the recombinant DNA having Bialaphos biosynthetic gene from these colonies was done according to the known hybridization method (Grunstein, M., Hogness, D.S., Proc. Natl. Acad. Sci. (U.S.A.), 72, 3961 - 3965, 1975). As probes, the pMSB 2-4 and pMSB 12-1 DNA labelled with $^{32}$P were employed, and hybridization was carried out according to the conditions of Bibb et al. (Nature, 284, 527 - 531, 1980). Colonies having the recombinant DNA reactive with both pMSB 2-4 and pMSB 12-1 probes were selected, and pMSB 13-3 was obtained therefrom.

Example 4 (Preparation of pMSB 1-2)

Streptomyces hygroscopicus SF-1293 was inoculated into 10 ml of S-I medium (2% soluble starch, 1.0% peptone, 0.3% meat extract, 0.05% $K_2HPO_4$, pH 7.0), cultured at 28°C for 24 to 48 hours, and the resultant culture broth was used as the seed microorganism and subinoculated into a MYG medium at a concentration of inoculated microorganism of 5%. The MYG medium was prepared by adding 2% glycine to 1% malt extract, 0.4 % yeast extract, 0.5% glucose, pH 7.0 (80 ml). This was subjected to shaking cultivation at 28°C for 24 hours, and the microorganism was collected by centrifugation at 10,000xg/10 min. From the microorganism cells, all DNA were extracted and purified according to the known method of Smith et al. (methods in Enzymology 12, 545, 1967) and dialyzed against a TESC buffer (10 mM Tris-HCl, pH 8.0, 1 mM EDTA, 2.5 mM NaCl) to produce a donor DNA.

The thus obtained donor DNA (10 µg) was incubated at 37°C for 2 hours with addition of 10 units of a restriction endonuclease BglII in a reaction medium of 230 µl total volume with a composition of 20 mM Tris-HCl, pH 7.4, 50 mM NaCl, 10 mM $MgCl_2$ and 10 mM β-meraptoethanol.

The plasmid pIJ702 DNA (2 µg) was digested at 37°C for 2 hours with 2 units of a restriction endonuclease BglII in a reaction medium of 40 µl total volume with a composition of 20 mM Tris-HCl, pH 7.4, 10 mM $MgCl_2$ and 10 mM β-meraptoethanol.

The reaction mixture was heated at 70°C for 10 minutes to deactivate the enzyme, then, with addition of 1/10 vol. 500 mM NaCl and 10 units of calf intestine alkaline phosphatase (c.i.a.p.), incubation was carried out at 37°C for 60 minutes.

To each of the reaction mixtures of the donor DNA and the plasmid DNA was added an equal volume of a phenol solution saturated with a TESH buffer (0.2 M Tris-HCl, pH 8.0, 20 mM dithiothreitol, 50 mM NaCl), then shaking was carried out for one minute, and the two mixtures were combined. After the combined mixture was centrifuged at 10,000xg/5 min., the upper layer (aqueous layer) was taken out with a Pasteur pipette.

The aqueous layer portion containing DNA was subjected to ethyl ether extraction to remove phenol, and an equal volume of ethanol was added. After the mixture was left to stand at -80°C for 2 hours, DNA was precipitated by centrifugation at 10,000xg/5 min.

The DNA thus obtained was washed with 500 µl of ethanol, dried under reduced pressure, and dissolved in 50 µl of heat sterilized pure water. The DNA was subjected to heat treatment at 70°C for 10 minutes, then cooled gradually to room temperature, and incubated with addition of 5 µl of a buffer (0.66 M Tris-HCl, pH 7.6, 66 mM $MgCl_2$, 0.1 M dithiothreitol, 10 mM ATP) and 5 µl (1 unit) of T4 ligase at 22°C for 2 hours to prepare a recombinant DNA of pIJ702 and the Streptomyces hygroscopicus DNA.

Transformation of the host microorganism Streptomyces hygroscopicus 369-IS strain with this DNA was carried out according to the protoplast PEG method conventionally used for actinomycetes (e.g. Curr. Topics Microbiol. Immunol., 96, 69, 1981).

More specifically, Streptomyces hygroscopicus 369-IS strain was inoculated into 80 ml of S medium (glucose 1%, peptone 0.4%, yeast extract 0.4%, $MgSO_4$. $7H_2O$ 0.05%, $KH_2PO_4$ 0.2%, $K_2HPO_4$ 0.4%, glycine 5%); shaking culture was carried out at 28°C for 18 hours; and mycelia was collected by centrifugation at 10,000xg, washed once with 0.5 M sucrose solution, and then suspended in 10 ml of Ps medium (NaCl 70 mM, sucrose 0.5 M, $MgCl_2$ 5 mM, $CaCl_2$ 5 mM, 25 mM TES pH 7.2). Then egg white lysozyme and Achromo-peptidase were added to obtain a final concentration of 1 mg/ml and 0.5 mg/ml, respectively, after which incubation was carried out at 37°C for 60 minutes to form protoplasts, and the mycelia not formed into protoplasts were removed by cotton filtration. The protoplasts were collected by centrifugation at 800xg/10 min., washed once with Ps medium, and suspended in 2 ml of Ps medium.

A mixture of 100 µl of the protoplast suspension, 100 µl of a P-maleate buffer adjusted to 3/2 concentration (2.5% sucrose, 1.4 mM $K_2SO_4$, 1/500 vol. trace elements solution, 100 mM $CaCl_2$, 50 ml Tris-maleate pH 8.0), 50 µl of the recombinant DNA solution and 375 µl of a polyethylene glycol solution (polyethylene glycol #1000, 33 %/P-maleate buffer) was left to stand at room temperature for one minute and then diluted with 5 ml of Ps medium, and the protoplasts were collected by centrifugation at 800xg/10 min. and suspended in 2 ml of Ps medium. After aliquots of the protoplast suspension, each in a quantity of 0.1 ml, were applied onto RH agar media prepared in 20 circular plastic petri dishes of 9 cm in diameter (containing, in one liter, sucrose 171 g, KCl 14.9 g, glucose 10 g, trace element solution 2 ml, 2.5% $K_2SO_4$ 10 ml, proline 3 g, casamino acid 0.5 g, polypeptone 2 g, dextran 0.05 g, 0.5% $KH_2PO_4$ 10 ml, 1M $CaCl_2 \cdot 2H_2O$ 50 ml, 0.25M TES (pH 7.2) 100 ml, CSC 30g/1000 ml 100 ml, 5% aspartic acid 10 ml), 2 ml of RH medium with an agar concentration of 0.5% was overlaid as the upper layer.

On this layer, after cultivation at 28°C for 18 hours, was further overlaid 2 ml of the same RH (soft agar 0.5%) with thiostrepton added thereto to 20 μg/ml. This was continued to be cultured at 28°C, in a chamber maintained at a humidity of 90%, for 10 to 20 days. The microorganism on the RH agar medium regenerated from protoplasts was transferred onto a nutrient agar medium containing 1% CAS, 5 μg/ml $CuSO_4 \cdot 5H_2O$ and 0.5 mg/ml thyrosine by means of a commercially available needle. This was subjected to culturing at 28°C for 2 days. As a result, colonies producing melanine dyes and colonies producing no melanine dye were formed in a ratio of about 1:1.

Since Streptomyces hygroscopicus DNA is incorporated at the BglII site on the gene participating in production of melanine of the vector plJ702, the clone introduced by the plasmid having foreign Streptomyces hygroscopicus DNA incorporated therein upon ligation will not produce melanine. However, the plasmid having no Streptomyces hygroscopicus DNA incorporated at the BglII site produces melanine dyes. Thus,the colonies which do not produce melanine dyes can be judged to have Streptomyces hygroscopicus DNA incorporated on the plasmid introduced.

Of these, the colonies producing no melanine dye was replicated onto the agar piece having a composition as shown below by use of a commercially available tooth-pick. The agar piece is a column of agar of 7 to 8 mm in diameter and 4 - 5 mm in height punched out with a cork borer from a composition comprising 0.4% glucose, 0.35% wheat germ, 0.23% SVP (soluble vegetable protein), 0.03% $KH_2PO_4$, 0.0001% $CoCl_2 \cdot 6H_2O$, 0.2% agar, pH 7.0.

The agar piece was subjected to cultivation at 28°C for 6 days. This was examined as to whether or not it was an agar piece forming an inhibitory circle at 32°C with the use of Proteus sp. MB-838 as the test microorganism. As a result, one strain of colony with the greatest diameter of the inhibitory circle was obtained.

The one strain of Streptomyces hygroscopicus obtained which was thiostrepton resistant was subjected to seed culturing similarly as described above and inoculated, in a quantity of 80 ml, into MYG medium (with added 2% glycine). From the microorganism cells after shaking cultivation at 28°C for 24 hours, the plasmid was extracted according to the known method by Hansen et al. (J. Bacteriol. 135, 227, 1978). When the strain 369-IS was transformed with these plasmids, the productivity of the strain containing pMSB 1-2 was found to be improved by 4- to 5-fold.

As to whether or not these microorganism strains produced Bialaphos, analysis was performed according to the method as disclosed in "Annual Research Report by Meiji Seika No. 21, 44 - 51, 1982". Briefly, the filtrate of each microorganism strain culture broth was analyzed with an amino acid analyzer, and the antimicrobial substance produced in the medium could be identified chromatographically as Bialaphos.

Example 5 (Preparation of pMSB 1-6)

Streptomyces hygroscopicus SF-1293 was inoculated into 10 ml of S-I medium (2% soluble starch, 1.0% peptone, 0.3% meat extract, 0.05% $K_2HPO_4$, pH 7.0), cultured at 28°C for 24 - 48 hours, and the resultant culture broth was used as the seed microorganism and subinoculated into a MYG medium at a concentration of inoculated microorganism of 5%. The MYG medium was prepared by adding 2% glycine to 1% malt extract, 0.4% yeast extract, 0.5% glucose, pH 7.0 (80 ml). This was subjected to shaking cultivation at 28°C for 24 hours, and the microorganism was collected by centrifugation at 10,000xg/10 min. From the microorganism cells, all DNA were extracted and purified according to the known method of Smith et al. (Methods in Enzymology 12, 545, 1967), and dialyzed against a TESC buffer (10 mM Tris-HCl, pH 8.0, 1 mM EDTA, 2.5 mM NaCl) to produce a donor DNA.

The thus obtained donor DNA (10 μg) was digested at 37°C for 2 hours with 10 units of a restriction endonuclease BglII in a reaction medium of 200 μl total volume with a composition of 20 mM Tris-HCl, pH 7.4, 50 mM NaCl, 10 mM $MgCl_2$ and 10 mM β-meraptoethanol.

The plasmid plJ702 DNA (2 μg) was digested at 37°C for 2 hours with 2 units of a restriction endonuclease BglII in a reaction medium of 40 μl total volume with a composition of 20 mM Tris-HCl, pH 7.4, 10 mM $MgCl_2$ and 10 mM β-meraptoethanol.

The reaction mixture was heated at 70°C for 10 minutes to deactivate the enzyme, then, with addition of 1/10 vol. 500 mM NaCl and 10 units of calf intestine alkaline phosphatase (c.i.a.p.), incubation was carried out at 37°C for 60 minutes.

To each of the reaction mixtures of the donor DNA and the plasmid DNA was added an equal volume of a phenol solution saturated with a TESH buffer (0.2 M Tris-HCl, pH 8.0, 20 mM dithiothreitol, 50 mM NaCl), which step was followed by shaking for one minute, and the two mixtures were combined. After the combined mixture was centrifuged at 10,000xg/5 min., the upper layer (aqueous layer) was taken out with a Pasteur pipette.

The aqueous layer portion containing DNA was subjected to ethyl ether extraction to remove phenol, and an equal volume of ethanol was added. After the mixture was left to stand at -80°C for 2 hours, DNA was precipitated by centrifugation at 10,000xg/5 min.

The DNA thus obtained was washed with 500 $\mu$l of ethanol, dried under reduced pressure and dissolved in 50 $\mu$l of heat-sterilized pure water. The DNA was subjected to heat treatment at 70°C for 10 minutes, then cooled gradually to room temperature and inculated with addition of 5 $\mu$l of a buffer (0.66 M Tris-HCl, pH 7.6, 66 mM $MgCl_2$, 0.1 M dithiothreitol, 10 mM ATP) and 5 $\mu$l (1 unit) of T4 ligase at 22°C for 2 hours to prepare a recombinant DNA of pIJ702 and the Streptomyces hygroscopicus DNA.

Transformation of the host microorganism Streptomyces hygroscopicus 369-IS strain with this DNA was carried out according to the protoplast PEG method conventionally used for actinomycetes (e.g., Curr. Topics Microbiol. Immunol., 96, 69, 1981).

More specifically, Streptomyces hygroscopicus 369-IS strain was inoculated into 80 ml of S medium (glucose 1%, peptone 0.4%, yeast extract 0.4%, $MgSO_4 \cdot 7H_2O$ 0.05%, $KH_2PO_4$ 0.2%, $K_2HPO_4$ 0.4%, glycine 5%); shaking culture was carried out at 28°C for 18 hours; and mycelia were collected by centrifugation at 10,000xg, washed once with 0.5 M sucrose solution, and then suspended in 10 ml of Ps medium (NaCl 70 mM, sucrose 0.5 M, $MgCl_2$ 5 mM, $CaCl_2$ 5 mM, 25 mM TES pH 7.2). Subsequently, egg white lysozyme was added to obtain a final concentration of 1 mg/ml and Achromo-peptidase to obtain a final concentration of 0.5 mg/ml, after which incubation was carried out at 37°C for 60 minutes to form protoplasts, and the mycelia not formed into protoplasts were removed by cotton filtration. The protoplasts were collected by centrifugation at 800xg/10 min., washed once with Ps medium, and suspended in 2 ml of Ps medium.

A mixture of 100 $\mu$l of the protoplast suspension, 100 $\mu$l of a P-maleic acid buffer adjusted to 3/2 concentration (2.5% sucrose, 1.4 mM $K_2SO_4$, 1/500 vol. trace elements solution, 100 mM $CaCl_2$, 50 ml Tris-maleic acid pH 8.0), 50 $\mu$l of the recombinant DNA solution and 375 $\mu$l of a polyethylene glycol solution (polyethylene glycol #1000, 33 %/P-maleate buffer) was left to stand at room temperature for one minute, then diluted with 5 ml of Ps medium, and the protoplasts were collected by centrifugation at 800xg/10 min. and suspended in 2 ml of Ps medium.

After aliquots of the protoplast suspension each of 0.1 ml were coated onto RH agar media prepared in 23 circular plastic petri dishes of 9 cm in diameter (containing, in one liter, sucrose 171 g, KCl 14.9 g, glucose 10 g, trace elements solution 2 ml, 2.5% $K_2SO_4$ 10 ml, proline 3 g, casamino acid 0.5 g, polypeptone 2 g, dextran 0.05 g, 0.5% $KH_2PO_4$ 10 ml, 1M $CaCl_2 \cdot 2H_2O$ 53 ml, 0.25M TES (pH 7.2) 100 ml, CSC 30g/1000 ml 100 ml, 5% aspartic acid 10 ml), 2 ml of RH medium with an agar concentration of 0.5% was overlaid as the upper layer. On this layer, after cultivation at 28°C for 18 hours, was further overlaid 2 ml of the same RH (soft agar 0.5%) with added thiostrepton to 20 $\mu$g/ml. This was continued to be cultured at 28°C, in a chamber maintained at a humidity of 90%, for 10 to 20 days.

The microorganism on the RH agar medium regenerated from protoplasts was transferred onto a nutrient agar medium containing 1% CAS, 5 $\mu$g/ml $CuSO_4 \cdot 5H_2O$ and 0.5 mg/ml thyrosine by means of a commercially available needle. This was subjected to culturing at 28°C for 2 days. As a result, colonies producing melanine dyes and colonies producing no melanine dye were formed in a ratio of about 1:1.

Since Streptomyces hygroscopicus DNA is incorporated at the BglII site on the gene participating in production of melanine of the vector pIJ702, the clone introduced by the plasmid having foreign Streptomyces hygroscopicus DNA incorporated therein upon ligation will not produce melanine. However, the plasmid having no Streptomyces hygroscopicus DNA incorporated at the BglII site produces melanine dyes. Thus, the colonies which do not produce melanine dyes can be judged to have Streptomyces hygroscopicus DNA incorporated on the plasmid introduced.

Of these, the colonies producing no melanine dye were replicated onto an agar piece having a composition as shown below by use of a commercially available tooth-pick. The agar piece is a column of agar of 7 to 8 mm in diameter and 4 - 5 mm in height punched out with a cork borer from a composition comprising 0.4% glucose, 0.35% wheat germ, 0.23% SVP (soluble vegetable protein), 0.03% $KH_2PO_4$, 0.0001% $CoCl_2 \cdot 6H_2O$, 2% agar, pH 7.0.

The agar piece was subjected to cultivation at 28°C for 6 days. This was examined as to whether or not it was an agar piece forming an inhibitory circle at 32°C with the use of Proteus sp. MB-838 as the test

14

microorganism. As a result, of 700 white colonies, one non-productive strain was obtained.

The one strain of Streptomyces hygroscopicus obtained which was thiostrepton resistant and yet lost its Bialaphos producing ability was subjected to seed culturing similarly as described above and inoculated, in quantities each of 80 ml, into MYG medium (with added 2% glycine). From the microorganism cells after shaking culture at 28°C for 24 hours, the plasmid pMSB 1-6 was extracted according to the known method by Hansen et al. (J. Bacteriol. 135, 227, 1978). When the Bialaphos producing strains such as Streptomyces hygroscopicus 369-IS strain and 460-2 strain were transformed again with these plasmids, each transformant was found to produce only a very minute amount of Bialaphos.

Example 6 (Preparation of pMSB 3-1)

Streptomyces hygroscopicus SF-1293 was inoculated into 10 ml of S-I medium (2% soluble starch, 1.0% peptone, 0.3% meat extract, 0.05% $K_2HPO_4$, pH 7.0), cultured at 28°C for 24 - 48 hours, and the resultant culture broth was used as the seed microorganism and subinoculated into a MYG medium at a concentration of inoculated microorganism of 5%. The MYG medium was prepared by adding 2% glycine to 1% malt extract, 0.4 % yeast extract, 0.5% glucose, pH 7.0 (80 ml). This was subjected to shaking cultivation at 28°C for 24 hours and the microorganism was collected by centrifugation at 10,000xg/10 min. From the microorganism cells, all DNA were extracted and purified according to the known method of Smith et al. (Methods in Enzymology 12, 545, 1967), and dialyzed against a TESC buffer (10 mM Tris-HCl, pH 8.0, 1 mM EDTA, 2.5 mM NaCl) to produce a donor DNA.

The thus obtained donor DNA (10 μg) was digested at 37°C for 2 hours with 10 units of a restriction endonuclease BglII in a reaction medium of 200 μl total volume with a composition of 20 mM Tris-HCl, pH 7.4, 50 mM NaCl, 10 mM $MgCl_2$ and 10 mM β-meraptoethanol.

The plasmid pIJ702 DNA (2 μg) was digested at 37°C for 2 hours with 2 units of a restriction endonuclease BglII in a reaction medium of 40 μl total volume with a composition of 20 mM Tris-HCl, pH 7.4, 10 mM $MgCl_2$ and 10 mM β-meraptoethanol.

The reaction mixture was heated at 70°C for 10 minutes to deactivate the enzyme, then, with addition of 1/10 vol. 500 mM NaCl and 10 units of calf intestine alkaline phosphatase (c.i.a.p.), incubation was carried out at 37°C for 60 minutes.

To each of the reaction mixtures of the donor DNA and the plasmid DNA was added an equal volume of a phenol solution saturated with a TESH buffer (0.2 M Tris-HCl, pH 8.0, 20 mM dithiothreitol, 50 mM NaCl), which step was followed by shaking for one minute, and the two mixtures were combined. After the combined mixture was centrifuged at 10,000xg/5 min., the upper layer (aqueous layer) was taken out with a Pasteur pipette.

The aqueous layer portion containing DNA was subjected to ethyl ether extraction of remove phenol, and an equal volume of ethanol was added. After the mixture was left to stand at -80°C for 2 hours, DNA was precipitated by centrifugation at 10,000xg/5 min.

The DNA thus obtained was washed with 500 μl of ethanol, dried under reduced pressure and dissolved in 50 μl of heat-sterilized pure water. The DNA was subjected to heat treatment at 70°C for 10 minutes, then cooled gradually to room temperature and incubated with addition of 5 μl of a buffer (0.66 M Tris-HCl, pH 7.6, 66 mM $MgCl_2$, 0.1 M dithiothreitol, 10 mM ATP) and 5 μl (1 unit) of T4 ligase at 22°C for 2 hours to prepare a recombinant DNA of pIJ702 and the Streptomyces hygroscopicus DNA.

Transformation of the host microorganism Streptomyces hygroscopicus NP51 strain with this DNA was carried out according to the protoplast PEG method conventionally used for actinomycetes (e.g., Curr. Topics Microbiol. Immunol., 96, 69, 1981).

More specifically, Streptomyces hygroscopicus NP51 strain was inoculated into 80 ml of S medium (glucose 1%, peptone 0.4%, yeast extract 0.4%, $MgSO_4 \cdot 7H_2O$ 0.05%, $KH_2PO_4$ 0.2%, $K_2HPO_4$ 0.4%, glycine 5%; shaking culture was carried out at 28°C for 18 hours; and mycelia were collected by centrifugation at 10,000xg, washed once with 0.5 M sucrose solution, and then suspended in 10 ml of Ps medium (NaCl 70 mM, sucrose 0.5 M, $MgCl_2$ 5 mM, $CaCl_2$ 5 mM, 25 mM TES pH 7.2). Then egg white lysozyme and Achromo-peptidase were added to obtain a final concentration of 1 mg/ml and 0.5 mg/ml, respectively, after which incubation was carried out at 37°C for 60 minutes to form protoplasts, and the mycelia not formed into protoplasts were removed by cotton filtration. The protoplasts were collected by centrifugation at 800xg/10 min., washed once with Ps medium, and suspended in 2 ml of Ps medium.

A mixture of 100 μl of the protoplast suspension, 100 μl of a P-maleic acid buffer adjusted to 3/2 concentration (2.5% sucrose, 1.4 mM $K_2SO_4$, 1/500 vol. trace elements solution, 100 mM $CaCl_2$, 50 ml Tris-maleic acid pH 8.0), 50 μl of the recombinant DNA solution and 375 μl of a polyethylene glycol solution (polyethylene glycol #1000, 33%/P-maleic acid buffer) was left standing at room temperature for one minute

then diluted with 5 ml of Ps medium, and the protoplasts were collected by centrifugation at 800xg/10 min. and suspended in 2 ml of Ps medium. After aliquots of the protoplast suspension each of 0.1 ml were coated onto RH agar media prepared in 20 circular plastic petri dishes of 90 cm in diameter (containing, in one liter, sucrose 171 g, KCl 14.9 g, glucose 10 g, trace element solution 2 ml, 2.5% $K_2SO_4$ 10 ml, proline 3 g, casamino acid 0.5 g, polypeptone 2 g, dextran 0.05 g, 0.5% $KH_2PO_4$ 10 ml, 1M $CaCl_2$ $2H_2O$ 50 ml, 0.25M TES (pH 7.2) 100 ml, CSC 30g/1000 ml 100 ml, 5% aspartic acid 10 ml), 2 ml of RH medium with an agar concentration of 0.5% was overlaid as the upper layer.

On this layer, after cultivation at 28°C for 18 hours, was further overlaid 2 ml of the same RH (soft agar 0.5%) with thiostrepton added to 20 $\mu$g/ml. This was continued to be cultured at 28°C, in a chamber maintained at a humidity of 90%, for 10 to 20 days. The microorganism on the RH agar medium regenerated from protoplasts was transferred onto a nutrient agar medium containing 1% CAS, 5 $\mu$g/ml $CuSO_4 \cdot 5H_2O$ and 0.5 mg/ml thyrosine by means of a commercially available needle. This was subjected to culturing at 28°C for 2 days. As a result, colonies producing melanine dyes and colonies producing no melanine dye were formed in a ratio of about 1:1.

Since Streptomyces hygroscopicus DNA is incorporated at the BglII site on the gene participating in production of melanine of the vector pIJ702, the clone introduced by the plasmid having foreign Streptomyces hygroscopicus DNA incorporated therein during ligation will not produce melanine. However, the plasmid having no Streptomyces hygroscopicus DNA incorporated at the BglII site produces melanine dyes. Thus, the colonies which do not produce melanine dyes can be judged to have Streptomyces hygroscopicus DNA incorporated on the plasmid introduced.

Of these, the colonies producing no melanine dye was replicated onto an agar piece having a composition as shown below by use of a commercially available tooth-pick. Agar piece is a column of agar of 7 to 8 mm in diameter and 4 - 5 mm in height punched out with a cork borer from a composition comprising 0.4% glucose, 0.35% wheat germ, 0.23% SVP (soluble vegetable protein), 0.03% $KH_2PO_4$, 0.0001% $CoCl_2 \cdot 6H_2O$, 2% agar, pH 7.0.

The agar piece was subjected to cultivation at 28°C for 6 days. This was examined as to whether or not it was an agar piece forming an inhibitory circle at 32°C with the use of Proteus sp. MB-838 as the test microorganism. As a result, of 1000 white colonies, one strain restored in productivity was obtained.

The one strain of Streptomyces hygroscopicus obtained which was thiostrepton resistant and yet restored in its Bialaphos producing ability was subjected to seed culturing similarly as described above and inoculated, in quantities each of 80 ml, into MYG medium (added with 2% glycine). From the microorganism cells after shaking culture at 28°C for 24 hours, the plasmid pMSB 1-6 was extracted according to the known method by Hansen et al. (J. Bacteriol. 135, 227, 1978). When Streptomyces hygroscopicus NP51 was transformed again with these plasmids, it could be restored in Bialaphos producing ability.

As to whether these microorganism strains produced Bialaphos or not, analysis was performed according to the method as disclosed in a Literature "Annual Research Report by Meiji Seika No.21, 44 - 51, 1982". Briefly, the filtrate of each microorganism strain culture broth was analyzed with an amino acid analyzer and the antimicrobial substance produced in the medium could be identified chromatographically as Bialaphos.

## Claims

1. A DNA strand comprising a gene coding for at least a part of the Bialaphos biosynthesis route, selected from the group consisting of the following DNA strands (1) through (6):

(1) a DNA strand (a) with the same base sequences as the DNA fragment derived from Bialaphos DNA or (b) with different base sequences due to degeneration of DNA codons having the same genotype as the strands under (a) in a hybrid plasmid pMSB 2-4 with a molecular weight of 9.15 kb having the restriction endonuclease cleavage site shown in Fig. 1, comprising a BamHI-digested fragment of Bialaphos DNA and a BglII-digested fragment of pIJ702 plasmid DNA;

(2) a DNA strand (a) with the same base sequences as the DNA fragment derived from Bialaphos DNA or (b) with different base sequences due to degeneration of DNA codons having the same genotype as the strands under (a) in a hybrid plasmid pMSB 12-1 with a molecular weight of 15.6 kb having the restriction endonuclease cleavage site shown in Fig. 2, comprising a SstI-digested fragment of Bialaphos DNA and a SstI-digested fragment of pIJ702 plasmid DNA;

(3) a DNA strand (a) with the same base sequences as the DNA fragment derived from Bialaphos DNA or (b) with different base sequences due to degeneration of DNA codons having the same genotype as the strands under (a) in a hybrid plasmid pMSB 13-3 with a molecular weight of 41 kb having the restriction endonuclease cleavage site shown in Fig. 3, comprising a Sau3AI-digested

fragment of Bialaphos DNA and a BamHI-digested fragment of cosmid pHC79 DNA;

(4) a DNA strand (a) with the same base sequences as the DNA fragment derived from Bialaphos DNA or (b) with different base sequences due to degeneration of DNA codons having the same genotype as the strands under (a) in a hybrid plasmid pMSB 1-2 with a molecular weight of 10.5 kb having the restriction endonuclease cleavage site shown in Fig. 4, comprising a BglII-digested fragment of Bialaphos DNA and a BglII-digested fragment of pIJ702 plasmid DNA;

(5) a DNA strand (a) with the same base sequences as the DNA fragment derived from Bialaphos DNA or (b) with different base sequences due to degeneration of DNA codons having the same genotype as the strands under (a) in a hybrid plasmid pMSB 1-6 with a molecular weight of 6.65 kb having the restriction endonuclease cleavage site shown in Fig. 5, comprising a BglII-digested fragment of Bialaphos DNA and a BglII-digested fragment of pIJ702 plasmid DNA; and

(6) a DNA strand (a) with the same base sequences as the DNA fragment derived from Bialaphos DNA or (b) with different base sequences due to degeneration of DNA codons having the same genotype as the strands under (a) in a hybrid plasmid pMSB 3-1 with a molecular weight of 14.7 kb having the restriction endonuclease cleavage site shown in Fig. 6, comprising a BglII-digested fragment of Bialaphos DNA and a BglII-digested fragment of pIJ702 plasmid DNA.

2. A plasmid pMSB 13-3 which has a molecular weight of 41 kb and has the restriction endonuclease cleavage site shown in Fig. 3.

3. The plasmid pMSB 13-3 as claimed in claim 2 which is included in E. coli K-12 LE392 (pMSB 13-3), FERM BP-853.

4. A transformant of E. coli which is E. coli K-12 LE 392 (pMSB 13-3), FERM BP-853.

## Revendications

1. Brin d'ADN comprenant un gène codant pour au moins une partie de la voie de biosynthèse du Bialaphos, choisi dans le groupe constitué par les brins d'ADN (1) à (6) suivants :

(1) un brin d'ADN (a) ayant les mêmes séquences de bases que le fragment d'ADN issu de l'ADN du Bialaphos ou (b) ayant des séquences de bases différentes en raison de la dégénérescence des codons d'ADN ayant le même génotype que les brins du point (a), dans un plasmide hybride pMSB 2-4 ayant une masse moléculaire de 9,15 kb ayant le site de clivage par une endonucléase de restriction représenté sur la Figure 1, comprenant un fragment digéré par BamHI de l'ADN du Bialaphos et un fragment digéré par BglII de l'ADN du plasmide pIJ702 ;

(2) un brin d'ADN (a) ayant les mêmes séquences de bases que le fragment d'ADN issu de l'ADN du Bialaphos ou (b) ayant des séquences de bases différentes en raison de la dégénérescence des codons d'ADN ayant le même génotype que les brins du point (a), dans un plasmide hybride pMSB 12-1 ayant une masse moléculaire de 15,6 kb ayant le site de clivage par une endonucléase de restriction représenté sur la Figure 2, comprenant un fragment digéré par SstI de l'ADN du Bialaphos et un fragment digéré par StsI de l'ADN du plasmide pIJ702 ;

(3) un brin d'ADN (a) ayant les mêmes séquences de bases que le fragment d'ADN issu de l'ADN du Bialaphos ou (b) ayant des séquences de bases différentes en raison de la dégénérescence des codons d'ADN ayant le même génotype que les brins du point (a), dans un plasmide hybride pMSB 13-3 ayant une masse moléculaire de 41 kb ayant le site de clivage par une endonucléase de restriction représenté sur la Figure 3, comprenant un fragment digéré par Sau3AI de l'ADN du Bialaphos et un fragment digéré par BamHI de l'ADN du cosmide pHC79 ;

(4) un brin d'ADN (a) ayant les mêmes séquences de bases que le fragment d'ADN issu de l'ADN du Bialaphos ou (b) ayant des séquences de bases différentes en raison de la dégénérescence des codons d'ADN ayant le même génotype que les brins du point (a), dans un plasmide hybride pMSB 1-2 ayant une masse moléculaire de 10,5 kb ayant le site de clivage par une endonucléase de restriction représenté sur la Figure 4, comprenant un fragment digéré par BglII de l'ADN du Bialaphos et un fragment digéré par BglII de l'ADN du plasmide pIJ702 ;

(5) un brin d'ADN (a) ayant les mêmes séquences de bases que le fragment d'ADN issu de l'ADN du Bialaphos ou (b) ayant des séquences de bases différentes en raison de la dégénérescence des codons d'ADN ayant le même génotype que les brins du point (a), dans un plasmide hybride pMSB 1-6 ayant une masse moléculaire de 6,65 kb ayant le site de clivage par une endonucléase de restriction représenté sur la Figure 5, comprenant un fragment digéré par BglII de l'ADN du Bialaphos et un fragment digéré par BgIII de l'ADN du plasmide pIJ702 ;

(6) un brin d'ADN (a) ayant les mêmes séquences de bases que le fragment d'ADN issu de l'ADN du Bialaphos ou (b) ayant des séquences de bases différentes en raison de la dégénérescence des codons d'ADN ayant le même génotype que les brins du point (a), dans un plasmide hybride pMSB 3-1 ayant une masse moléculaire de 14,7 kb ayant le site de clivage par une endonucléase de restriction représenté sur la Figure 6, comprenant un fragment digéré par BgIII de l'ADN du Bialaphos et un fragment digéré par BgIII de l'ADN du plasmide pIJ702 ;

2. Plasmide pMSB 13-3 qui a une masse moléculaire de 41 kb et a le site de clivage par une endonucléase de restriction représenté sur la Figure 3.

3. Plasmide pMSB 13-3 selon la revendication 2, qui est inclus dans E. coli K-12 LE 392 (pMSB 13-3), FERM BP-853.

4. Transformant d'E. coli qui est E. coli K-12 LE 392 (pMSB 13-3), FERM BP-853.

## Patentansprüche

1. Ein DNA-Strang umfassend ein Gen mit einer Codierung für mindestens einen Teil der Bialaphos-Biosyntheseroute, ausgewählt aus der Gruppe bestehend aus den folgenden DNA-Strängen(1) bis (6):

(1)ein DNA-Strang(a) mit der gleichen Basensequenz wie das aus der Bialaphos-DNA abgeleitete DNA-Fragment oder (b) mit davon verschiedenen Basensequenzen aufgrund der Degeneration von DNA-Kodierungseinheiten(Codone) mit dem gleichen Genotypus wie die Stränge (a) in einem hybriden Plasmid pMSB 2-4 mit einem Molekulargewicht von 9,15 kb mit der in Fig.1 dargestellten Restriktions-Endonuklease-Abspaltstelle, umfassend ein BamHi-digestiertes Fragment von Bialaphos-DNA und ein BglII-digestiertes Fragment von pIJ702-Plasmid-DNA;

(2)ein DNA-Strang(a) mit der gleichen Basensequenz wie das aus der Bialaphos-DNA abgeleitete DNA-Fragment oder (b) mit davon verschiedenen Basensequenzen aufgrund der Degeneration von DNA-Kodierungseinheiten mit dem gleichen Genotypus wie die Stränge (a) in einem hybriden Plasmid pMSB 12-1 mit einem Molekulargewicht von 15,6 kb mit der Restriktions-Endonuklease-Abspaltstelle, wie in Fig.2 dargestellt, umfassend ein SstI-digestiertes Fragment von Bialaphos-DNA und ein SstI-digestiertes Fragment von pIJ702-Plasmid-DNA;

(3)ein DNA-Strang(a) mit der gleichen Basensequenz wie das aus der Bialaphos-DNA abgeleitete DNA-Fragment oder (b) mit davon verschiedenen Basensequenzen aufgrund der Degeneration von DNA-Kodierungseinheiten mit dem gleichen Genotypus wie die Stränge (a) in einem hybriden Plasmid pMSB 13-3 mit einem Molekulargewicht von 41 kb mit der in Fig.3 dargestellten Restriktions-Endonuklease-Abspaltstelle, umfassend ein Sau3AI-digestiertes Fragment von Bialaphos-DNA und ein BamHi-digestiertes Fragment von Cosmid-phC79-DNA;

(4)ein DNA-Strang(a) mit der gleichen Basensequenz wie das aus der Bialaphos-DNA abgeleitete DNA-Fragment oder (b) mit davon verschiedenen Basensequenzen aufgrund der Degeneration von DNA-Kodierungseinheiten mit dem gleichen Genotypus wie die Stränge (a) in einem hybriden Plasmid pMSB 1-2 mit einem Molekulargewicht von 10,5 kb mit der in Fig.4 dargestellten Restriktions-Endonuklease-Abspaltstelle, umfassend ein BglII-digestiertes Fragment von Bialaphos DNA und ein BglII-digestiertes Fragment von pIJ702-Plasmid-DNA;

(5)ein DNA-Strang(a) mit der gleichen Basensequenz wie das aus der Bialaphos-DNA abgeleitete DNA-Fragment oder (b) mit davon verschiedenen Basensequenzen aufgrund der Degeneration von DNA-Kodierungseinheiten mit dem gleichen Genotypus wie die Stränge (a) in einem hybriden Plasmid pMSB 1-6 mit einem Molekulargewicht von 6,65 kb mit der in Fig.5 dargestellten Restriktions-Endonuklease-Abspaltstelle, umfassend ein BglII-digestiertes Fragment von Bialaphos DNA und ein BglII-digestiertes Fragment von pIJ702-Plasmid-DNA; und

(6) ein DNA-Strang(a) mit der gleichen Basensequenz wie das aus der Bialaphos-DNA abgeleitete DNA-Fragment oder (b) mit davon verschiedenen Basensequenzen aufgrund der Degeneration von DNA-Kodierungseinheiten mit dem gleichen Genotypus wie die Stränge (a) in einem hybriden Plasmid pMSB 3-1 mit einem Molekulargewicht von 14,7 kb mit der in Fig.6 dargestellten Restriktions-Endonuklease-Abspaltstelle, umfassend ein BglII-digestiertes Fragment von Bialaphos-DNA und ein BglII-digestiertes Fragment von pIJ702-Plasmid-DNA.

2. Ein Plasmid pMSB 13-3, welches ein Molekulargewicht von 41 kb und die in Fig.3 dargestellte Restriktions-Endonuklease-Abspaltstelle aufweist.

3. Das Plasmid pMSB 13-3 wie in Anspruch 2 beansprucht, welches in E.Coli K-12 LE392(pMSB 13-3), FERM BP-853 enthalten ist.

4. Ein Transformant von E.coli, welcher in E.coli K-12 LE 392(pMSB 13-3), FERM BP-853 enthalten ist.

# FIG. 1

pMSB 2-4
9.15 kb

# FIG. 2

pMSB 12-1
15.6 kb

# FIG. 3

pMSB 13-3
41 kb

# FIG. 4

pMSB 1-2
10.5 kb

# FIG. 5

pMSB 1-6
6.65kb

# FIG. 6

pMSB 3-1
14.7 kb

22

# FIG. 7

EP 0 173 327 B1

FIG. 8

EP 0 173 327 B1